# EUROPEAN PATENT APPLICATION

(11) **EP 1 688 511 A1**
(43) Date of publication of application: **09.08.2006**
(21) Application number: 05075260.9
(22) Date of filing: 02.02.2005
(51) Int. Cl.: C22C 38/00, C22C 38/44, C07C 273/00

(54) **Process for the production of urea in a conventional urea plant**

(71) Applicant: DSM IP Assets B.V., 6411 TE Heerlen (NL)
(72) Inventor: Mennen, Johannes Henricus, 5768 XN Meijel (NL); Jonckers, Kees, 6116 AK Roosteren (NL)
(74) Representative: Scheltus, Irma

(57) **Abstract**

The invention is directed to a process for the production of urea in a conventional urea plant comprising a high-pressure part, which comprises a urea reactor wherein the urea reactor comprises an austenitic-ferritic duplex steel with a chromium content of between 25 and 35 wt.% and a nickel content of between 3 and 10 wt. %.

## Description

The invention is directed to a process for the production of urea in a conventional urea plant comprising a high-pressure part, which comprises a urea reactor.

Urea is produced nowadays in two types of urea plants; conventional urea plants and urea stripping plants. Conventional urea plants were developed before the urea stripping plants. Conventional urea plants and urea stripping plants are, for instance, described in Ullmann's Encyclopedia of Industrial Chemistry, Vol. A 27, 1996, p. 339 - 350.

A conventional urea plant is defined by the fact that the ammonium carbamate streams that are returned to the urea reactor have a pressure that is lower than the pressure in the urea reactor. The pressure employed in the high-pressure part of the urea plant, comprising the urea reactor, is 15-25 MPa. The urea solution formed in the urea reactor is lowered in pressure directly after the reactor and is further treated in a medium-pressure recirculation stage, operating at a pressure of 1.8-2.5 MPa, and thereafter in a low-pressure recirculation stage, operating at a pressure of 0.2-0.5 MPa. A urea stripping plant comprises, in the high-pressure part of the plant, normally a reactor, a stripper, a condenser and optionally a scrubber.

The pressure in the reactor of a conventional urea plant is about 5 MPa higher than the pressure in the reactor of a urea stripping plant. Because of this high pressure often corrosion and leakage problems occur in the reactor of a conventional urea plant. This even after passivation air is added to the reactor to prevent corrosion.

The object of the present invention is to overcome these problems.

The invention is characterized in that the urea reactor comprises an austenitic-ferritic duplex steel with a chromium content of between 25 and 35 wt.% and a nickel content of between 3 and 10 wt.%.

Austenitic-ferritic duplex steels show a very good corrosion resistance in an environment comprising ammonium carbamate. Ammonium carbamate is an intermediate product in the production of urea and is very corrosive. It has now been found that austenitic-ferritic duplex steel with a chromium content of between 25 and 35 wt.% and a nickel content of between 3 and 10 wt.% have an even better corrosion resistance in an environment comprising ammonium carbamate. This means that less passivation of the steel is necessary. As less less passivation air is needed when using the duplex steel according to the invention, it is achieved that the reactor in a conventional urea plant can be operated at a lower pressure, This results in less corrosion and leakage problems; herewith prolonging substantially the lifetime of the reactor and the safety of the urea plant. By using a lower pressure in the reactor also the energy used to raise the pressure of the ammonia and/or the carbon dioxide feed streams is lower, resulting in lower operation costs for the urea plant.

Preferably, the parts in the urea reactor that are in contact with the reaction mixture are made of an austenitic-ferritic duplex steel with a chromium content of between 25 and 35 wt.% and a nickel content of between 3 and 10 wt.%. When this is the case, the amount of passivation air needed can be lowered even more and the pressure in the high-pressure part of the conventional urea plant can be lower.

More preferably, the the liner and the internals in the urea reactor are made of the austenitic-ferritic duplex steel with a chromium content of between 25 and 35 wt.% and a nickel content of between 3 and 10 wt.%.

Urea is normally produced out of ammonia and carbon dioxide as starting products, but it is also possible to use an ammonium carbamate containing stream as a reactor feed.

A process for the production of urea using, at least partially, an ammonium carbamate containing feed stream is for instance described in US-4,433,146. According to this patent publication an ammonium carbamate containing stream originating from a melamine plant is, together with additional ammonia, fed to a urea reactor after it has been preheated. In this urea plant the high-pressure part of the plant comprises not only the urea reactor, but also the heater. According to the invention it is advantageous that the heater comprises an austenitic-ferritic duplex steel with a chromium content of between 25 and 35 wt.% and a nickel content of between 3 and 10 wt.%.

More preferably, the parts in the heater that are in contact with the reaction mixture are made of an austenitic-ferritic duplex steel with a chromium content of between 25 and 35 wt.% and a nickel content of between 3 and 10 wt.%. Most preferably, the pipes in the heater are made of the austenitic-ferritic duplex steel with a chromium content of between 25 and 35 wt.% and a nickel content of between 3 and 10 wt.%.

Preferably the chromium content in the austenitic-ferritic duplex steel is between 26 and 30 wt.%; more preferably between 26 and 28 wt%.

Preferably the nickel content in the austenitic-ferritic duplex steel is between 5 and 10 wt.%; more preferably between 5 and 8 wt%.

Most preferably an austenitic-ferritic duplex steel with the following composition is used:

| | |
|---|---|
| C: | maximum 0.05 wt.% |
| Si : | maximum 0.8 wt.% |
| Mn: | 0.3-4.0 wt.% |
| Cr : | 25 - 35 wt.% |
| Ni : | 3 - 10 wt.% |
| Mo : | 0.2 - 4.0 wt.% |
| N : | 0.2-0.6 wt.% |
| Cu : | maximum 1.0 wt.% |
| W : | maximum 3.0 wt.% |
| S : | maximum 0.01 wt.% |
| Ce : | maximum 0.2 wt.%. |

the balance consisting of Fe and common impurities and additives.

The invention is also directed to a conventional urea plant comprising a high pressure part comprising a urea reactor, wherein the reactor comprises an austenitic-ferritic duplex steel with a chromium content of between 25 and 35 wt.% and a nickel content of between 3 and 10 wt.%.

Preferably, for the same reason as described above, the parts that are in contact with the reaction mixture are made of an austenitic-ferritic duplex steel with a chromium content of between 25 and 35 wt.% and a nickel content of between 3 and 10 wt.%. More preferably, the liner and the internals in the urea reactor are made of the austenitic-ferritic duplex steel with a chromium content of between 25 and 35 wt.% and a nickel content of between 3 and 10 wt.%.

The invention is also directed to a conventional urea plant, wherein the urea plant is, at least partially, fed with a stream containing ammonium carbamate that is preheated in a heater before entering the urea reactor, wherein the heater comprises an austenitic-ferritic duplex steel with a chromium content of between 25 and 35 wt.% and a nickel content of between 3 and 10 wt.%.

Preferably, the parts in the heater that are in contact with the reaction mixture are made of an austenitic-ferritic duplex steel with a chromium content of between 25 and 35 wt.% and a nickel content of between 3 and 10 wt.%. More preferably, the pipes in the heater are made of the austenitic-ferritic duplex steel with a chromium content of between 25 and 35 wt.% and a nickel content of between 3 and 10 wt.%.

The invention also covers a urea reactor, wherein the reactor comprises an austenitic-ferritic duplex steel with a chromium content of between 25 and 35 wt.% and a nickel content of between 3 and 10 wt.%.

Preferably, the parts in the urea reactor that are in contact with the reaction mixture are made of an austenitic-ferritic duplex steel with a chromium content of between 25 and 35 wt.% and a nickel content of between 3 and 10 wt.%.

More preferably, the liner and the internals in the urea reactor are made of an austenitic-ferritic duplex steel with a chromium content of between 25 and 35 wt.% and a nickel content of between 3 and 10 wt.%.

## Claims

1. Process for the production of urea in a conventional urea plant comprising a high-pressure part, which comprises a urea reactor, **characterized in that** the urea reactor comprises an austenitic-ferritic duplex steel with a chromium content of between 25 and 35 wt.% and a nickel content of between 3 and 10 wt.%.

2. Process according to claim 1, **characterized in that** parts in the urea reactor that are in contact with the reaction mixture are made of an austenitic-ferritic duplex steel with a chromium content of between 25 and 35 wt.% and a nickel content of between 3 and 10 wt.%.

3. Process according to claim 1 or 2, wherein the urea reactor comprises a liner and internals, **characterized in that** the liner and the internals in the urea reactor are made of an austenitic-ferritic duplex steel with a chromium content of between 25 and 35 wt.% and a nickel content of between 3 and 10 wt.%.

4. Process according to any one of claims 1-3, wherein the urea reactor is, at least partially, fed with a stream containing ammonium carbamate that is preheated in a heater before entering the urea reactor, **characterized in that** the heater comprises an austenitic-ferritic duplex steel with a chromium content of between 25 and 35 wt.% and a nickel content of between 3 and 10 wt.%.

5. Process according to any one of claims 1-4, **characterized in that** parts in the heater that are in contact with the reaction mixture are made of an austenitic-ferritic duplex steel with a chromium content of between 25 and 35 wt.% and a nickel content of between 3 and 10 wt.%.

6. Process according claim 5, wherein the heater comprises pipes, **characterized in that** the pipes in the heater are made of an austenitic-ferritic duplex steel with a chromium content of between 25 and 35 wt.% and a nickel content of between 3 and 10 wt.%.

7. Process according to any one of claims 1-6, **characterized in that** austenitic-ferritic duplex steel with the following composition is used:
| | |
|---|---|
| C: | maximum 0.05 wt.% |
| Si : | maximum 0.8 wt.% |
| Mn : | 0.3 - 4.0 wt.% |
| Cr : | 25 - 35 wt.% |
| Ni : | 3 - 10 wt.% |
| Mo : | 0.2-4.0 wt.% |
| N | : 0.2-0.6 wt.% |
| Cu : | maximum 1.0 wt.% |
| W : | maximum 3.0 wt.% |
| S : | maximum 0.01 wt.% |
| Ce : | maximum 0.2 wt.%. |
the balance consisting of Fe and common impurities and additives.

8. Conventional urea plant comprising a high pressure part comprising a urea reactor, **characterized in that** the reactor comprises an austenitic-ferritic duplex steel with a chromium content of between 25 and 35 wt.% and a nickel content of between 3 and 10 wt.%.

9. Conventional urea plant according to claim 8, **characterized in that** parts in the urea reactor that are in contact with the reaction mixture are made of an austenitic-ferritic duplex steel with a chromium content of between 25 and 35 wt.% and a nickel content of between 3 and 10 wt.%.

10. Conventional urea plant according to claim 8 or 9, wherein the urea reactor comprises a liner and internals, **characterized in that** the liner and the internals in the urea reactor are made of an austenitic-ferritic duplex steel with a chromium content of between 25 and 35 wt.% and a nickel content of between 3 and 10 wt.%.

11. Conventional urea plant according to any one of claims 8-10, wherein the urea plant is, at least partially, fed with a stream containing ammonium carbamate that is preheated in a heater before entering the urea reactor, **characterized in that** the heater comprises an austenitic-ferritic duplex steel with a chromium content of between 25 and 35 wt.% and a nickel content of between 3 and 10 wt.%.

12. Conventional urea plant according to claim 11, **characterized in that** parts in the heater that are in contact with the reaction mixture are made of an austenitic-ferritic duplex steel with a chromium content of between 25 and 35 wt.% and a nickel content of between 3 and 10 wt.%.

13. Conventional urea plant according claim 11 or 12, wherein the heater comprises pipes, **characterized in that** the pipes in the heater are made of an austenitic-ferritic duplex steel with a chromium content of between 25 and 35 wt.% and a nickel content of between 3 and 10 wt.%.

14. Urea reactor, **characterized in that** the reactor comprises an austenitic-ferritic duplex steel with a chromium content of between 25 and 35 wt.% and a nickel content of between 3 and 10 wt.%.

15. Urea reactor according to claim 14, **characterized in that** parts in the urea reactor that are in contact with the reaction mixture are made of an austenitic-ferritic duplex steel with a chromium content of between 25 and 35 wt.% and a nickel content of between 3 and 10 wt.%.

16. Urea reactor according to claim 14 or 15, wherein the urea reactor comprises a liner and internals, **characterized in that** the liner and the internals in the urea reactor are made of an austenitic-ferritic duplex steel with a chromium content of between 25 and 35 wt.% and a nickel content of between 3 and 10 wt.%.
